# EUROPEAN PATENT APPLICATION

(11) **EP 1 935 900 A1**
(43) Date of publication of application: **25.06.2008**
(21) Application number: 08004058.7
(22) Date of filing: 05.12.2001
(51) Int. Cl.: C07K 14/47, A23L 1/305, A61K 38/17, C07K 16/18

(54) **Peptides derived from colostrinin**

(30) Priority: 06.12.2000 GB 0029777
(62) Divisional of application: 01999572.9
(71) Applicant: ReGen Therapeutics Plc, London EC4M 9BJ (GB)
(72) Inventor: Georgiades, Jerzy A., Houston,TX 77063 (US)
(74) Representative: Curtis, Philip Anthony

(57) **Abstract**

The amino acid sequence of several peptides is disclosed. These peptides are useful, inter alia, in the treatment of disorders of the immune system and the central nervous system, and are also useful as food additives.

SEQUENCE LISTING

<110>  ReGen Therapeutics plc

<120>  Peptides Derived from Colostrinin

<130>  20307A

<150>  GB0029777.0
<151>  2000-12-06

<160>  17

<170>  PatentIn version 3.3

<210>  1
<211>  19
<212>  PRT
<213>  Ovis aries

<400>  1

Leu Val Tyr Pro Phe Thr Gly Pro Ile Pro Asn Ser Leu Pro Gln Asn
1               5                   10                  15

Ile Leu Pro

<210>  2
<211>  13
<212>  PRT
<213>  Ovis aries

<400>  2

Met Ile Val Val Arg Leu Leu Gln Asn Glu Val Pro Glu
1               5                   10

<210>  3
<211>  10
<212>  PRT
<213>  Ovis aries

<400>  3

Ser Leu Ser Gln Ser Lys Val Leu Pro Val
1               5                   10

<210>  4
<211>  8
<212>  PRT
<213>  Ovis aries

<400>  4

Leu Gln Thr Gln Thr Pro Val Val
1               5


<210>  5
<211>  7
<212>  PRT
<213>  Ovis aries

<400>  5

Glu Met Pro Phe Pro Lys Tyr
1               5


<210>  6
<211>  6
<212>  PRT
<213>  Ovis aries

<400>  6

Pro Val Glu Pro Phe Thr
1               5


<210>  7
<211>  6
<212>  PRT
<213>  Ovis aries

<400>  7

Val Pro Pro Phe Leu Gln
1               5


<210>  8
<211>  5
<212>  PRT
<213>  Ovis aries

<400>  8

Pro Met Phe Leu Gln
1               5


<210>  9
<211>  5
<212>  PRT
<213>  Ovis aries

<400>  9

Glu His Met Phe Val
1               5


<210>  10
<211>  4
<212>  PRT
<213>  Ovis aries

<400>  10

Thr Asp Arg Asp
1


<210>  11
<211>  4
<212>  PRT
<213>  Ovis aries

<400>  11

Val Gln Pro Thr
1


<210>  12
<211>  4
<212>  PRT
<213>  Ovis aries

<400>  12

Pro Lys Val Lys
1


<210>  13
<211>  4
<212>  PRT
<213>  Ovis aries

<400>  13

Asp Asp Asp Glu
1


<210>  14
<211>  4
<212>  PRT
<213>  Ovis aries

<400>  14

Thr Glu Glu Val
1

<210> 15
<211> 4
<212> PRT
<213> Ovis aries

<400> 15

Tyr Gln Gln Glu
1

<210> 16
<211> 4
<212> PRT
<213> Ovis aries

<400> 16

Phe Pro Pro Gln
1

<210> 17
<211> 4
<212> PRT
<213> Ovis aries

<400> 17

Gly Phe Gly Ile
1

## Description

The present invention relates to peptides. More particularly the invention relates to certain peptides isolated from Colostrinin. The invention also relates to therapeutic uses of the peptides and to antibodies derived therefrom.

Colostrum is the thick, yellowish fluid produced by a mammalian mother's breasts during the first few days after childbirth. It is the first lacteal secretion post parturition and it contains a high concentration of immunoglobulins (IgG, IgM and IgA) and other proteins. It is replaced by mature breast milk about four to five days after birth. Compared with mature breast milk, colostrum contains low sugar and iron, but is rich in lipids, proteins, mineral salts, vitamins and immunoglobulins. Colostrum also contains various floating cells such as granular and stromal cells, neutrophils, monocyte/macrophages and lymphocytes and includes growth factors, hormones, cytokines and polypeptide complexes.

Various factors have been isolated and characterised from mammalian colostrum. In 1974, Janusz et al (FEBS Lett., 49, 276-279) isolated a proline-rich polypeptide (PRP) from ovine colostrum. It has since been discovered that mammals other than sheep have analogues of PRP as a component of their colostrum. PRP has since been called Colostrinin (and is sometimes called Colostrinine).

M. Janusz & J. Lisowski in "Proline-Rich Polypeptide (PRP) - an Immunomodulatory Peptide from Ovine Colostrum" (Archivum Immunologiae et Therapiae Experimentalis, 1993, 41, 275-279) mentioned that PRP from ovine colostrum has immunotropic activity in mice.

A. Dubowska-Inglot et al in "Colostrinine: a proline-rich polypeptide from ovine colostrum is a modest cytokine inducer in human leukocytes" (Archivum Immunologiae et Therapiae Experimentalis, 1996, 44, 215-224) discussed the use of Colostrinin in the treatment of Alzheimer's disease. The use of Colostrinin in the treatment of Alzheimer's disease, and other conditions, was also discussed in WO-A-98/14473 and in "Colostrinin: a Proline-Rich Polypeptide (PRP) Complex Isolated from Ovine Colostrum for Treatment of Alzheimer's Disease. A Double-Blind, Placebo-Controlled Study", Leszek,J. et al, Archivum Immunologiae et Therapiae Experimentalis, 1999, 47, 377-385.

Colostrinin, in its natural form, is obtained from mammalian colostrum. As described in WO-A-98/14473, analysis by electrophoresis and chromatography has shown that Colostrinin has the following properties:
(i) it has a molecular weight in the range 16,000 to 26,000 Daltons (this was shown by electrophoresis in the presence of SDS);
(ii) it is a dimer or trimer of sub-units each sub-unit having a molecular weight in the range 5,000 to 10,000 Daltons (this was shown by acrylamide gel electrophoresis in the presence of SDS);
(iii) it contains proline, and the amount of proline is greater than the amount of any other single amino acid (this can be shown by conventional amino acid analysis).

By means of these techniques it was shown that ovine Colostrinin has a molecular weight of about 18,000 Daltons, is made up of three non-covalently linked sub-units each having a molecular weight of about 6,000 Daltons and includes about 22 wt% proline. The amino-acid composition of ovine Colostrinin was shown to be made up of the following number of residues per sub-unit: lysine - 2, histidine - 1, arginine - 0, aspartic acid - 2, threonine - 4, serine - 3, glutamic acid - 6, proline - 11, glycine - 2, alanine - 0, valine - 5, methionine - 2, isoleucine - 2, leucine - 6, tyrosine - 1, phenylalanine - 3 and cysteine - 0.

In our international patent publication no. WO00/75173 we further analysed the composition of Colostrinin in order to try to identify its components, so that a synthetic form of Colostrinin can be produced.

The invention provides peptides containing or consisting of one of the amino acid sequences: LVYPFTGPIPNSLPQNILP (SEQ. ID 1); MIWRLLQNEVPE (SEQ. ID 2); SLSQSKVLPV (SEQ. ID 3); LQTQTPW (SEQ. ID 4); EMPFPKY (SEQ. ID 5); PVEPFT (SEQ. ID 6); VPPFLQ (SEQ. ID 7); PMFLQ (SEQ. ID 8); EHMFV (SEQ. ID 9); TDRD (SEQ. ID 10); VQPT (SEQ. ID 11); PKVK (SEQ. ID 12); DDDE (SEQ. ID 13); TEEV (SEQ. ID 14); YQQE (SEQ. ID 15); FPPQ (SEQ. ID 16); GFGI (SEQ. ID 17); LQS (SEQ. ID 18); VW (SEQ. ID 19); GGK (SEQ. ID 20); DMV (SEQ. ID 21); ESQ (SEQ. ID 22); GRV (SEQ. ID 23); VEE (SEQ. ID 24); IGN (SEQ. ID 25); FFQ (SEQ. ID 26); RMF (SEQ. ID 27); FPP (SEQ. ID 28); MHH (SEQ. ID 29); NTE (SEQ. ID 30).

These peptides may be provided in substantially isolated form. They may be formed by a synthetic process. Furthermore, a composition may be provided which contains two or more of the above peptides, in combination.

in respect of the peptides 1 to 30, the invention further includes any peptide which includes the specified amino acid sequence. In respect of the peptides 1 to 30, the invention further comprises any peptide which includes an amino-terminal amino acid sequence corresponding to the specified sequence. Thus, with reference to peptide 1, for example, the invention encompasses any peptide having the N-terminal amino acid sequence LVYPFTGGPIPNSLPQNILP; the same applies to peptides 2 to 30. For the avoidance of doubt, it is stated that the amino-terminal end is on the left hand side of the sequence, in accordance with the usual convention. It will be appreciated that any of the specified amino acid sequences may be provided with an inert amino acid sequence on the amino-terminal and/or the carboxy-terminal end thereof. The invention further includes physiologically acceptable active derivatives of the peptides.

The peptides were identified using the methods described in examples 1 and 2 of WO00/75173, the contents of which are incorporated herein by reference.

The peptides described in WO/0075173 were described as falling into four categories, Group A (peptides of unknown precursor), Group B (peptides [possibly] having beta-casein homologue precursor), Group C (peptides having beta-casein precursor) and Group D (peptides having annexin precursor). Of the above peptides, nos. 2, 3 and 4 appear to fall into group A, peptides 1 and 28 appear to fall into group B, peptides 5 to 17 appear to fall into group C, and peptides 18 to 27, 29 and 30 appear to fall into a group E which contains peptides too small to be classified.

The peptides can be obtained by a number of techniques. In one embodiment, they can be prepared naturally by isolation from Colostrinin or colostrum. In a preferred embodiment, they are prepared by a conventional technique for peptide synthesis, such as by solid-phase or liquid-phase peptide synthesis. Alternatively, the gene sequence encoding the peptides can be constructed by known techniques such as expression vectors or plasmids and transfected into suitable microorganisms that will express the DNA sequences, whereby the peptides can be later extracted from the medium in which the microorganisms are grown. Thus, the invention also embraces a DNA sequence encoding the peptides described above, and a recombinant vector prepared by inserting said DNA in a vector.

The peptides, either alone or in combination with one another, have a number of therapeutic uses.

In one advantageous embodiment, one or more of peptides 1 to 30 may be used in the treatment of disorders of the central nervous system, particularly chronic disorders of the central nervous system. The disorders of the central nervous system that may be treated include neurological disorders and mental disorders. Examples of neurological disorders that may, with advantage, be treated include dementia, and also disorders that cause dementia, such as neurodegenerative disorders. Neurodegenerative disorders include, for example, senile dementia and motor neurone disease; Parkinson's disease is an example of a motor neurone disease that can be treated. Alzheimer's disease is an example of a neurodegenerative disease that can be treated. Examples of mental disorders that can be treated by one or more of the peptides include psychosis and neurosis. For example, the peptides may.be used to treat emotional disturbances, especially the emotional disturbances of psychiatric patients in a state of depression. The peptides may also be used as an auxiliary withdrawal treatment for drug addicts, after a period of detoxification, and in persons dependent on stimulants.

In another advantageous embodiment of the invention, one or more of peptides 1 to 30 may be used in the treatment of disorders of the immune system, particularly chronic disorders of the immune system the may occur spontaneously in people of advanced age. The peptides can also be used in the treatment of diseases requiring immuno-modulation. The peptides are useful in the treatment of a variety of diseases with an immunological and infectious basis. For example, they can be used to treat chronic diseases with a bacterial and viral aetiology, and to treat acquired immunological. deficiencies that have developed, for example, after chemotherapy or radiotherapy of neoplasms. The peptides may be used for treating chronic bacterial and viral infections requiring non-specific immunostimulation and immunocorrection.

A chronic disorder is a disorder that has persisted, or is expected to persist, for a long time, i.e., at least 3 months and usually at least 6 months.

One or more of the peptides may be used for improving the development of the immune system of a new born child. It is a further feature of the invention to use the peptides to correct immunological deficiencies in a child. These uses of the peptides may be particularly applicable to babies or children who have been deprived of colostrum. This may occur, for example, in babies and children who were not breast fed from birth.

The peptides, either alone or in combination with one another, also have diagnostic and research applications. For example, the synthetic peptides, as well as the corresponding antibodies described below, may be used to recognise pathological processes occurring in a host. These processes may be induced by excessive production or inhibition of the peptides or the antibodies. Once the pathological process, associated with a particular level of the peptides or the antibodies is known, measuring the production of the peptides and the antibodies in body fluids may be used to determine pathological processes taking place in the host.

According to another aspect of the invention, we provide the use of one or more of peptides 1 to 30 as a dietary supplement. This dietary supplement is particularly useful for babies, especially premature babies and babies at term, and for young children to correct deficiencies in the development of their immune system. The dietary supplement may also be used as a dietary supplement for adults, including senile persons, who have been subjected to chemotherapy, or have suffered from cahexia, or weight loss due to chronic disease.

In an aspect of the invention, we provide a dietary supplement comprising an orally ingestible combination of one or more of peptides 1 to 30 in combination with a physiologically acceptable carrier. The dietary supplement may be provided in liquid or solid form; the dietary supplement may suitably be provided in the form of a tablet. The dietary supplement may be provided in the form of a baby food formula. The dietary supplement may include, as an additive, lactoferrin and/or selenium and/or a group of cytokines containing members of the interferon family.

In accordance with the invention, one or more of peptides 1 to 30 may be administered prophylactically in order to help to prevent the development of disorders of the central nervous system and the immune system.

The peptides according to the invention may be used to promote the dissolution of -amyloid plaques, and, therefore, the peptides may be used in the treatment of any disease which is characterised by the development of -amyloid plaques.

The peptides according to the invention may be administered in a dosage in the range 1 ng to 10 mg. A dosage unit of about 3 µg is typical. However, the optimum dosage will, of course, depend upon the condition being treated.

The peptides according to the invention may be formulated for administration in any suitable form. Thus, the invention further provides a composition, especially a pharmaceutical composition, which includes one or more of the peptides in combination with a physiologically acceptable carrier. The peptides may, for example, be formulated for oral, topical, rectal or parenteral administration. More specifically, the peptides may be formulated for administration by injection, or, preferably, in a form suitable for absorption through the mucosa of the oral/nasopharyngeal cavity, the alimentary canal or any other mucosal surface. The peptides may be formulated for administration intravenously, subcutaneously, or intramuscularly. The oral formulations may be provided in a form for swallowing or, preferably, in a form for dissolving in the saliva, whereby the formulation can be absorbed in the mucous membranes of the oral/nasopharyngeal cavity. The oral formulations may be in the form of a tablet for oral administration, lozenges (i.e. a sweet-like tablet in a form suitable to be retained in the mouth and sucked), or adhesive gels for rubbing into the gum. The peptides may be formulated as an adhesive plaster or patch, which may be applied to the gums. The peptides may also be formulated for application to mucous-membranes of the genito-urinary organs. The topical formulations may be provided in the form of, for example, a cream or a gel.

One or more of the peptides may be incorporated into products like milk or cheese spread.

We have found that the ratio of the peptides in colostrum varies over time. Owing to hormonal changes, many proteins secreted into colostrum become sequentially degraded. The longer the time from parturition the more extensive the degradation can be. This knowledge will help with the design of new baby food formulas as well as' many drugs for immuno-compromised patients.

In another aspect, the invention provides an antibody for each of the peptides 1 to 30, and provides compositions containing said antibodies. In particular the invention provides the antibodies in substantially isolated form. The antibodies can be produced by injecting a suitable mammalian subject, such as a rabbit, with the corresponding peptide (with a suitable adjuvant), then recovering the antibodies from the subject after allowing time for them to be produced. This technique is described in detail in Example 3. It is possible to test that the correct antibody has been produced by ELISA (enzyme-linked immunosorbent assay) using the synthetic peptides as antigens. The antibodies can be further tested against the natural peptides in Colostrinin as confirmation that the synthetic peptides do correspond to the natural peptides found in Colostrinin. The antibodies have potential uses in therapy, as a diagnostic tool and as a research tool. The antibodies can be produced in accordance with the methods described in example 3 of WO00/75173.

The invention also encompasses the selective administration of one or more of peptides 1 to 30, at selected times to a patient, and the selective administration of one or more of the antibodies for the peptides in order to switch on or off the activity of the peptides at a selected time.

A selection of selected ones of the peptides and/or antibodies may be provided in a single composition which is specially tailored to produce a particular effect. For example, for a person with an immunological disorder, the composition can be specially tailored for that disorder. The composition may be specially selected for more than one disorder. The composition may be specially selected to restore or produce a particular balance in a subject.

In some applications it may be desirable to provide a pharmaceutical composition which contains one or more of the peptides and one or more of the antibodies in combination with a physiologically acceptable carrier.

The invention further embraces the use of one or more of the peptides and/or antibodies in the manufacture of a medicament for use in any of the therapeutic applications described above.

It will be appreciated that the invention described above may be modified.

## Claims

1. A peptide which substantially includes the amino-terminal amino acid sequence: SLSQSKVLPV (SEQ. ID 3);

2. A peptide which substantially entirely consists of the amino acid sequence: SLSQSKVLPV (SEQ. ID 3);

3. A peptide according to claim 1 or 2 in substantially isolated form.

4. A peptide according to claim 1, 2 or 3, when obtained by a synthetic process.

5. A peptide according to any preceding claim, for use as a medicament.

6. A peptide according to claim 5, for use in the treatment of chronic disorders of the central nervous system.

7. A peptide according to claim 5, for use in the treatment of neurological disorders and/or mental disorders.

8. A peptide according to claim 5, for use in the treatment of dementia and/or neurodegenerative diseases.

9. A peptide according to claim 5, for use in the treatment of Alzheimer's disease and/or motor neurone disease.

10. A peptide according to claim 5, for use in the treatment of psychosis and/or neurosis.

11. A peptide according to claim 5, for use in the treatment of chronic disorders of the immune system.

12. A peptide according to claim 5, for use in the treatment of diseases with a bacterial and viral aetiology, and/or for use in the treatment of acquired immunological deficiencies.

13. A peptide according to claim 5, for use in the treatment of chronic bacterial and/or viral infections.

14. A peptide according to claim 5, for use in the treatment of diseases **characterised by** the presence of-amyloid plaque.

15. The use of a peptide according to any one of claims 1 to 4, in the manufacture of a medicament for the treatment of chronic disorders of the central nervous system.

16. The use of a peptide according to any one of claims 1 to 4 in the manufacture of a medicament for the treatment of chronic disorders of the immune system.

17. A method of treating disorders of the central nervous system and/or of the immune system, comprising administering a therapeutically effective amount of a peptide according to any one of claims 1 to 4 a patient.

18. A pharmaceutical composition comprising a peptide according to any one of claims 1 to 4, in combination with a physiologically acceptable carrier.

19. A composition comprising two or more peptides according to any one of claims 1 to 4, in combination with a physiologically acceptable carrier.

20. A pharmaceutical composition according to claim 18 or 19, in a form suitable for injection.

21. A pharmaceutical composition according to claim 18 of 19, in a form suitable for absorption through the mucosa of the oral/nasopharyngeal cavity and/or in a form suitable for absorption in the alimentary canal.

22. A composition according to claim 18 or 19, in a form suitable for tropical application.

23. The use of a peptide according to any one of claims 1 to 4 as a dietary supplement.

24. The use of a peptide according to any one of claims 1 to 4 as a dietary supplement.

25. The use of a peptide according to any one of claims 1 to 4 as a dietary supplement for babies, small children, adults who have been subjected to chemotherapy and/or adults who have suffered from cahexia, or weight loss due to chronic disease.

26. A dietary supplement comprising an orally ingestible combination of a peptide according to any one of claims 1 to 4 in combination with a physiologically acceptable carrier.

27. An antibody which binds to a peptide according to any one of claims 1 or 4.

28. An antibody obtainable by using a peptide according to any one of claims 1 to 4 as an antigen.
